# EUROPEAN PATENT APPLICATION

(11) **EP 4 039 299 A1**
(43) Date of publication of application: **10.08.2022**
(21) Application number: 21218267.9
(22) Date of filing: 30.12.2021
(51) Int. Cl.: A61M 15/00, A61M 11/00, A61M 15/08

(54) **DRUG DELIVERY DEVICE, METHOD OF CONTROLLING FLUID PLUME AND METHOD OF NASAL CAVITY INJECTION**

(30) Priority: 04.02.2021 US 202117167409
(71) Applicant: Funai Electric Co., Ltd., Daito, Osaka, 574-0013 (JP)
(72) Inventor: GIBSON, Bruce D., Lexington, KY 40508 (US); JONES, Brian T., Lexington, KY 40508 (US); MARRA III, Michael A., Lexington, KY 40508 (US); MILGATE III, Robert W., Lexington, KY 40508 (US)
(74) Representative: Becker Kurig & Partner Patentanwälte mbB

(57) **Abstract**

A device (200) includes a cartridge body; a fluid outlet nozzle (208) attached thereto; and a cartridge disposed therein. A fluid ejection head (202) is attached to the cartridge and is in fluid flow communication with the fluid outlet nozzle (208). The cartridge contains a liquid pharmaceutical drug. A logic board is disposed in cartridge body and is electrically connected to the ejection head (202). A processor is disposed on the logic board or on the fluid ejection head (202) for executing a control algorithm to control the ejection head (202) to modify plume characteristics of fluid ejected from the ejection head (202) by controlling parameters selected from (a) a number of fluid ejectors (214, 216, 218, 220, 222) fired per ejection burst, (b) a position of fluid ejectors (214, 216, 218, 220, 222) fired per ejection burst, and (c) both (a) and (b).

## Description

### TECHNICAL FIELD

The disclosure is directed to inhalation drug delivery systems and in particular to controlling plume characteristics of fluid jet drug delivery systems for inhalation applications.

### BACKGROUND AND SUMMARY

Nasal spay devices have become important methods for delivering drugs to patients. Such nasal spray devices are more convenient to use than the administration of drugs through IV or injection. Nasal spray devices also provide higher bioavailability of drugs compared to oral administration of drugs. The absorption of drugs through nasal spray devices is more rapid compared to the absorption of drugs administered orally since drugs delivered by nasal spray devices directly enter the blood stream making their effect more immediate.

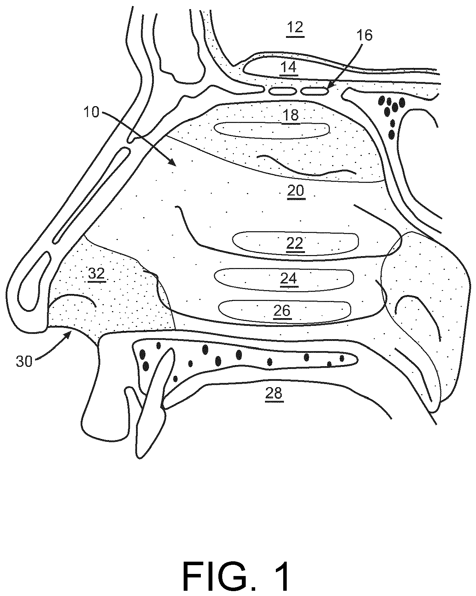

FIG. 1 is a cross sectional view, not to scale, of anatomy of a nasal cavity 10 of a person. A portion of the brain 12 is shown above the nasal cavity 10. An olfactory bulb 14 is disposed between the brain 12 and a cribriform plate 16. An olfactory mucosa 18 is below the cribriform plate 16. The nasal cavity also includes a superior turbinate 20, a middle turbinate 22, respiratory mucosa 24 and an inferior turbinate 26. Item 28 represents the palate. Injection of a pharmaceutical drug mist enters the nasal cavity 10 through the nostrils 30 and squamous mucosa 32. In order to achieve proper delivery of drugs to the blood stream using a nasal spray device, the drugs must be delivered to the respiratory mucosa which is highly vascularized. Two areas that are highly vascularized are the olfactory region and the respiratory region. The respiratory region contains turbinates which increase the surface area available for drug absorption.

It is believed that smaller, lower velocity fluid droplets are best for deposition of drugs in the nasal cavity. Fluid droplets with high inertia will tend to move in a straight line and land at the point only where they are aimed. Fluid droplets with low inertia will be affected by air resistance and air currents and are more likely to float throughout the nasal cavity for more even drug delivery coverage.

Another apsect of nasal delivery of drugs that may increase deposition coverage is the plume angle of the fluid droplets. A wider plume angle is believed to provide greater mist formation and thus better coverage of drug delivery in the nasal cavity. Conventional methods for delivering drugs via the nasal cavity include medicine droppers, multi-spray bottles with spray tips, single-dose syringes with spray tips, and dry powder systems. Accordingly, conventional drug delivery devices are typically designed to deliver a specific drug to a nasal cavity and each device cannot be adapted for delivering a wide range of drugs via a nasal cavity route. Many of the conventional methods for nasal drug delivery rely on pressurized containers to inject a mist of fluid into the nasal cavity. Accordingly, the drug delivery devices are typically designed for a specific drug and cannot be adapted to administer a different drug.

Despite the availability of a variety of devices for delivering drugs via a nasal cavity route, there remains a need for a single nasal drug delivery device that can be tuned to deliver a variety of drugs over a range of velocities, fluid ejection times, and plume angles.

In view of the foregoing, an embodiment of the disclosure provide a pharmaceutical drug delivery device and method of using the pharmaceutical drug delivery device.

In one embodiment, the pharmaceutical drug deliver device includes a cartridge body; a fluid outlet nozzle attached to the cartridge body; a fluid jet ejection cartridge containing a liquid pharmaceutical drug disposed in the cartridge body. A fluid ejection head is attached to the fluid jet ejection cartridge and the fluid ejection head is in fluid flow communication with the fluid outlet nozzle. A processor is disposed on a logic board or on the fluid ejection head for executing a control algorithm to control the ejection head to modify plume characteristics of fluid ejected from the ejection head by controlling one or more of an operating parameter selected from (a) a number of fluid ejectors fired per ejection burst, (b) a position of fluid ejectors fired per ejection burst, and (c) both (a) and (b).

In another embodiment, there is provided a method of controlling a fluid plume from a fluid ejection device for delivery of pharmaceutical drugs The method includes providing a cartridge body for the fluid ejection device, a fluid outlet nozzle attached to the cartridge body and a fluid jet ejection cartridge containing a pharmaceutical drug disposed in the cartridge body. A fluid ejection head is attached to the fluid jet ejection cartridge and the fluid ejection head is in fluid flow communication with the fluid outlet nozzle. A processor is provided in electrical communication with the fluid ejection head. The processor is configured to execute a control algorithm to select one or more operating parameters selected from (a) a number of fluid ejectors fired per ejection burst, (b) a position of fluid ejectors fired per ejection burst, and (c) both (a) and (b) in order to modify fluid plume characteristics of fluid ejected from the ejection head through the fluid outlet nozzle. The fluid ejection device is activated to deliver the pharmaceutical drug to a patient.

In another embodiment, there is provided a method for nasal cavity injection of pharmaceutical drugs. The method includes providing a fluid ejection device containing a cartridge body, a fluid outlet nozzle attached to the cartridge body and a fluid jet ejection cartridge containing a pharmaceutical drug disposed in the cartridge body. A fluid ejection head is attached to the fluid jet ejection cartridge and the fluid ejection head is in fluid flow communication with the fluid outlet nozzle. A processor is provided in electrical communication with the fluid ejection head. The processor is configured to execute a control algorithm to select one or more operating parameters selected from (a) a number of fluid ejectors fired per ejection burst, (b) a position of fluid ejectors fired per ejection burst, and (c) both (a) and (b) in order to modify fluid plume characteristics of fluid ejected from the ejection head through the fluid outlet nozzles. The fluid ejection device is activated to deliver the pharmaceutical drug in the nasal cavity of a person.

In some embodiments, each fluid droplet ejected from the ejection head has volume ranging from about 2 to about 24 pL.

In some embodiments, the ejection head has a firing frequency ranging from about 10 KHz to about 20 KHz.

In some embodiments, the number of fluid ejectors fired per ejection burst ranges from about 40 to about 200 fluid ejectors per burst.

In some embodiments, the pharmaceutical drug is delivered to the patient with a fluid plume angle ranging from about 25 to about 60 degrees.

In some embodiments, the pharmaceutical drug is delivered to the patient with a fluid plume height ranging from about 15 to about 25 centimeters.

In some embodiments, the pharmaceutical drug is delivered to the patient with a fluid jet length ranging from about 2 to about 10 centimeters from the fluid ejection head.

In some embodiments, the pharmaceutical drug is ejected with a plume characteristic that delivers the drug to a mucosa area of a nasal cavity of the patient.

In some embodiments, the pharmaceutical drug is ejected with a plume characteristic that evenly distributes the drug throughout a nasal cavity of the patient.

In some embodimens, the pharmaceutical drug is ejected with a plume characteristic that increases a drug dose delivery rate to the patient.

An advantage of the pharmaceutical drug delivery device described herein is that the device may be used for a wide variety of drugs having different fluid characteristics. The device is tunable by modifying certain fluid ejector operating parameters in order to modify a plume angle, fluid jet length, and/or plume height of fluid mist for nasal injection applications. Other features and advantage of the disclosed embodiments may be evident from the following drawings and detailed description.

### BRIEF DESCRIPTION OF THE DRAWINGS

FIG. 1 is a cross-sectional representation, not to scale, of a portion of a nasal cavity and skull of a person.
FIG. 2 is a cross-sectional view, not to scale of a pharmaceutical drug delivery device according to an embodiment of the disclosure.
FIG. 3 is a schematic illustration, not to scale, of a fluid ejection device showing a fluid ject and fluid plume generated by the fluid ejection device.
FIG. 4 is a plan view, not to scale, of an ejection head for the pharmaceutical drug delivery device of FIG. 2, that may be operated according to embodiments of the disclosure.
FIG. 5 is a schematic illustration, not to scale, of the fluid ejection device of FIG. 3 having fluid plume and fluid jet characteristics when non-adjacent fluid ejectors are activated.
FIG. 6 is a schematic illustration, not to scale, of the fluid ejection device of FIG. 3 having fluid plume and fluid jet characteristics when adjacent fluid ejectors are activated.
FIG. 7 is a plan view, not to scale, of an ejection head for the pharmaceutical drug delivery device of FIG. 2, that may be operated according to another embodiment of the disclosure.

### DETAILED DESCRIPTION

For the purposes of this disclosure, the following terms are defined:
a) plume means the randomly directed mist of fluid droplets with low inertia that are affected by air resistance and air currents and are likely to float throughout a nasal chamber for more even coverage;
b) plume angle is a measure of an angle of a cone-shaped volume of randomly directed mist of fluid droplets in the plume;
c) plume height is a measure of a total height of mist of fluid droplets in a plume measured from an outlet of a fluid ejection head to a total travel distance of the plume;
d) fluid jet length is a measure of a length of high inertia fluid droplets ejected from an outlet of an ejection head to the apex of the plume angle;
e) burst is defined as the number of times a fluid droplet is ejected from an individual nozzle. A burst of fluid occurs when a fluid ejector is fired by a series of voltage pulses of sufficient magnitude to eject fluid through an associated nozzle;
f) burst length is defined as the total number of times each of the fluid ejectors is fired per burst; and
g) burst delay is defined as amount of time between individual bursts.

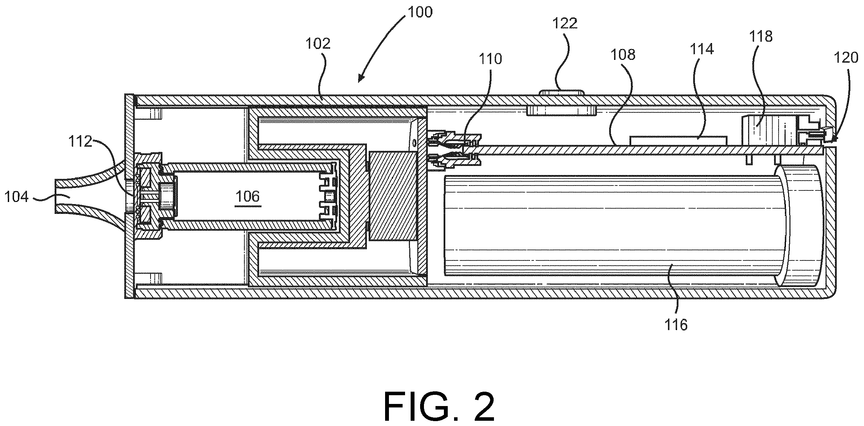

An illustration of a pharmaceutical drug delivery device 100 is illustrated in a cross-sectional view, not to scale, in FIG. 2. The device includes a a cartridge body 102, having a fluid outlet nozzle 104 attached to the cartridge body 102. A fluid jet ejection cartridge 106 is disposed in the cartrdige body 102. The fluid jet ejection cartridge 106 contains a liquid pharmaceutical drug to be administered by the device 100. A logic board 108 is disposed in cartridge body 102 and is electrically connected via a logic board connector 110 to an ejection head 112 on the fluid jet ejection cartridge 106. A processor 114 is disposed on the logic board 108 or on the ejection head 112 for executing a control algorithm to control the ejection head 112 to modify plume characteristics of fluid ejected from the ejection head by controlling one or more operating parameters selected from (a) a number of fluid ejectors fired per ejection burst, (b) a position of fluid ejectors fired per ejection burst, and (c) both (a) and (b). The cartridge body 102 may include a rechargeable battery 116 electrically connected to the logic board 108 for providing power to the ejection head 112. A power switch 118 is used to activate the device 100. A USB input 120 may also be included to reprogram the processor for use with different pharmaceutical fluids. An activation button 122 may be used to initiate delivery of the pharmaceutical drug on-demand by a user.

A wide variety of ejection heads 112 may be used with the device 100 described above. Accordingly, the ejection head 112 may be selected from a thermal jet ejection head, a bubble jet ejection head or a piezoelectric jet ejection head. Each of the foregoing ejection heads can produce a spray of fluid on demand and may be programmed to provide a variety of fluid plume characteristics as described below. By contrast, conventional spray pumps are mechanically fixed for a particular drug delivery application and generally cannot be modified to provide a variety of fluid plume characteristics.

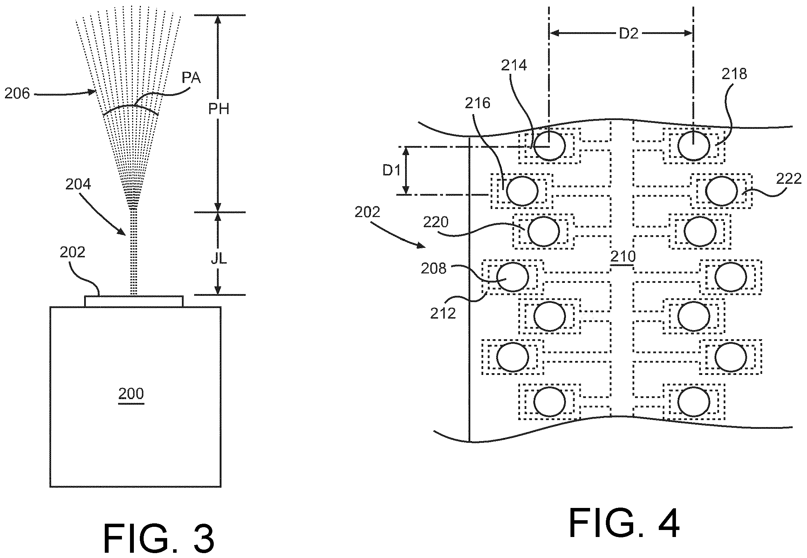

Unlike conventional inkjet ejection heads which are designed to eject fluid droplets in a straight line for 2 to 3 mm to reach a substrate such as paper, the device 100 described herein is designed to eject fluid droplets as a mist further into an air stream so that the droplets eventually land in the mucosa area of the nasal cavity. FIG. 3 is a schematic illustration of fluid jet ejection device 200 having an ejection head 202 for ejecting fluid therefrom to form a relatively high velocity fluid jet stream 204 and a low velocity plume 206 of fluid mist that floats on ambient air currents. The plume 206 characteristics, such as plume height PH and plume angle PA as well as the fluid jet stream length JL may be affected by how the ejection head 202 is operated. For example, the number of fluid ejectors activated per fluid ejection burst can be used to affect the fluid jet length JL, plume height PH and plume angle PA. Likewise, the position of fluid ejectors fired per ejection burst can be used to affect the fluid jet length JL, plume height PH, and plume angle PA of fluid ejected from the device 100. In some embodiments, the number of fluid ejectors fired per ejection burst and the position of fluid ejectors fired per ejection burst may be use to affect the fluid jet length JL, plume height PH and plume angle PA. A wider plume 206 may be the result of collisions between droplets as they are ejected from the ejection head 202. Another characteristics that effects the plume 206 is the entrainment of fluid droplets from the ejection head 202. High velocity fluid droplets tend to create an airflow perpendicular to the ejection head 202 which entrains subsequent droplets ejected from the ejection head 202 to draw the droplets further from the ejection head 202. Accordingly, air entrainment can affect both the fluid jet stream 204 and the plume 206 characteristics.

A portion of the ejection head 202 is illustrated in plan view in FIG. 4. The ejection head 202 contains a nozzle plate containing a plurality of fluid outlet nozzles 208 wherein each fluid outlet nozzle 208 is disposed above a fluid ejector. In some embodiments, the ejection head contains from about 100 to about 400 nozzles 208. Each nozzle 208 is sized to expell from about 2 to about 24 pL per droplet. In some embodiments, each nozzle 208 is sized to expell about 4 pL per droplet. Fluid to be ejected through the fluid outlet nozzles 208 is supplied from a fluid cartridge through a fluid via 210 to an ejection chamber 212 containing the fluid ejector. For the purposes of this disclosure, adjacent fluid ejectors are ejectors 216 and 218 with respect to ejector 214. Fluid ejectors 214 is spaced from fluid ejector 216 a first distance D1. Fluid ejector 214 is spaced from fluid ejector 218 a second distance D2.

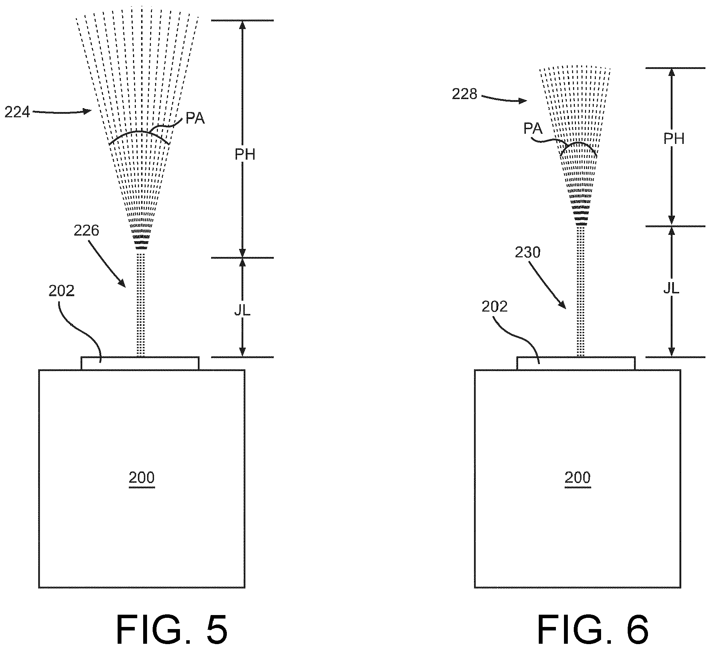

Accordingly, in one embodiment, a "position" of fluid ejectors and associated nozzles 208 may be referred to as adjacent to one another or may be spaced-apart or non-adjacent to one another. Non-adjacent nozzles 208, such as fluid ejectors 220 and 222 with respect to fluid ejector 214, may be activated at the same time to eject fluid from the associated nozzles 208, rather than activating adjacent fluid ejectors 216 and 218 with respect to fluid ejector 214. Thus, for ejection head 202, adjacent fluid ejectors 216 and 218 with respect to fluid ejector 214 may be skipped during an ejection burst. For example, an ejection head having 96 fluid fluid ejectors and associated nozzles 208, may be programmed to activate only 48 fluid ejectiors out of 96 fluid ejectors (48 per side of the fluid via 210) per ejection burst at a frequency of 18 KHz to provide an fluid plume 224 and fluid jet 226 as shown in FIG. 5. The fluid plume 224 of FIG. 5 has a greater plume angle PA and has a greater plume height PH than the fluid plume of an ejection head 202 having 96 fluid ejectors (48 per side of the fluid via 210) operating at 18 KHz wherein adjacent fluid ejectors are activated. FIG. 6 illustrates a fluid plume 228 and fluid jet 230 for an ejection head wherein adjacent fluid ejectors are activated resulting in a narrower plume 228, smaller plume angle PA and shorter plume height PH than the fluid plume 224 of FIG. 5. The results of firing all 96 fluid ejectors versus skipping every other fluid ejector are shown in the following table.

**Table**

| **Nozzle/ejector position** | **Firing Frequency (KHz)** | **# of Fluid Ejectors Fired per burst** | **Single Fire pulse (ns)** | **Voltage (V)** | **Preheat Temperature (°C)** | **PA (°)** | **Plume width (cm)** | **PL (cm)** | **JL (cm)** |
|---|---|---|---|---|---|---|---|---|---|
| 48 nozzles on each side of fluid via 210 (fire non-adjacent fluid ejectors) | 18 | 48 | 800 | 11 | 45 | 35 | 4.0 | 19.5 | 4.0 |
| 48 nozzles on each side of fluid via 210 (fire adjacent fluid ejectors) | 18 | 96 | 800 | 11 | 45 | 25 | 3.5 | 19.0 | 4.5 |

Thus a single fluid ejection head 202 having 96 fluid ejectors can be operated in multiple fashions to obtain different plume characteristics allowing the same ejection head to be used for multiple applications. For example, adjacent fluid ejectors can be activated for applications that require a maximum flow rate of fluid to be ejected. The same ejection head 202 can be programmed to activate non-adjacent fluid ejectors for alternate applications, such as nose to brain drug delivery requiring a minimum flow rate of fluid to be ejected. When non-adjacent fluid ejectors are activated, the dose delivery rate is reduced and the time to deliver a predetermined dose of pharmaceutical drug is increased.

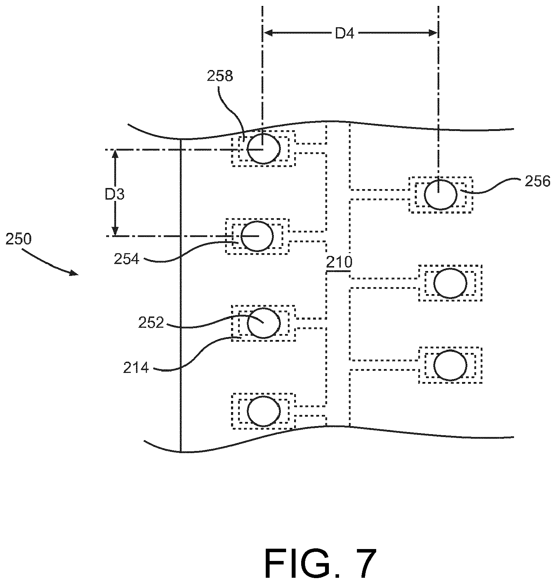

In another embodiment, a larger ejection head 250 (FIG. 7) containing 96 fluid ejectors and associated nozzles 252 may be used. In this embodiment, adjacent fluid ejectors, such as fluid ejectors 254 and 256 may be spaced-apart from one another such that distances D3 and D4 from fluid ejector 258 are greater than the distances D1 and D2 shown in FIG. 4. In this case, all 96 fluid ejectors (48 per side of the fluid via 210) may be activated at the same time resulting in plume characteristics similar to the plume characteristics of the ejection head 202 wherein non-adjacent fluid ejectors are activated. However, since all 96 fluid ejectors are activated at the same time, the dose delivery rate can be maintained at a higher rate than when non-adjacent fluid ejectors are activated. It will be appreciated that the ejection head 250 can also be programmed to eject fluid from non-adjacent nozzles as described above for lower drug dose delivery rates. Thus, the foregoing embodiments enable a pharmaceutical drug delivery device to be tune or programmed to provide multiple plume characteristics for delivery of drugs to a particular area of the nasal cavity without having to provide a unique ejection head for multiple pharmaceutical drug applications. The foregoing embodiments also provide an ability to tune the fluid plume for a particular application without the need for external mechanisms to control plume characteristics.

It is noted that, as used in this specification and the appended claims, the singular forms "a," "an," and "the," include plural referents unless expressly and unequivocally limited to one referent. As used herein, the term "include" and its grammatical variants are intended to be nonlimiting, such that recitation of items in a list is not to the exclusion of other like items that can be substituted or added to the listed items

For the purposes of this specification and appended claims, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that can vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

While particular embodiments have been described, alternatives, modifications, variations, improvements, and substantial equivalents that are or can be presently unforeseen can arise to applicants or others skilled in the art. Accordingly, the appended claims as filed and as they can be amended are intended to embrace all such alternatives, modifications variations, improvements, and substantial equivalents.

## Claims

1. A pharmaceutical drug delivery device (100, 200) comprising:
a cartridge body (102);
a fluid outlet nozzle (104, 208, 252) attached to the cartridge body (102);
a fluid jet ejection cartridge (106) containing a liquid pharmaceutical drug is disposed in the cartridge body (102), wherein a fluid ejection head (112, 202, 250) is attached to the fluid jet ejection cartridge (106) and the fluid ejection head (112, 202, 250) is in fluid flow communication with the fluid outlet nozzle (104, 208, 252); and
a processor (114) disposed on a logic board (108) or on the fluid ejection head (112, 202, 250) for executing a control algorithm to control the fluid ejection head (112, 202, 250) to modify plume characteristics of fluid ejected from the fluid ejection head (112, 202, 250) by controlling one or more of an operating parameter selected from the group consisting of (a) a number of fluid ejectors (214, 216, 218, 220, 222, 254, 256, 258) fired per ejection burst, (b) a position of fluid ejectors (214, 216, 218, 220, 222, 254, 256, 258) fired per ejection burst, and (c) both (a) and (b).

2. The pharmaceutical drug delivery device (100, 200) of claim 1, wherein each fluid droplet ejected from the fluid ejection head (112, 202, 250) has volume ranging from about 2 to about 24 pL.

3. The pharmaceutical drug delivery device (100, 200) of claim 1, wherein the fluid ejection head (112, 202, 250) has a firing frequency ranging from about 10 KHz to about 20 KHz.

4. The pharmaceutical drug delivery device (100, 200) of claim 1, wherein the number of fluid ejectors (214, 216, 218, 220, 222, 254, 256, 258) fired per ejection burst ranges from about 40 to about 200 fluid ejectors (214, 216, 218, 220, 222, 254, 256, 258) per burst.

5. A method of controlling a fluid plume from a fluid ejection device (100, 200) for delivery of pharmaceutical drugs, the method comprising:
providing a cartridge body (102) for the fluid ejection device (100, 200), a fluid outlet nozzle (104, 208, 252) attached to the cartridge body (102) and a fluid jet ejection cartridge (106) containing a pharmaceutical drug disposed in the cartridge body (102), wherein a fluid ejection head (112, 202, 250) is attached to the fluid jet ejection cartridge (106) and the fluid ejection head (112, 202, 250) is in fluid flow communication with the fluid outlet nozzle (104, 208, 252),
providing a processor (114) in electrical communication with the fluid ejection head (112, 202, 250),
configuring the processor (114) to execute a control algorithm to select one or more operating parameters selected from the group consisting of (a) a number of fluid ejectors (214, 216, 218, 220, 222, 254, 256, 258) fired per ejection burst, (b) a position of fluid ejectors (214, 216, 218, 220, 222, 254, 256, 258) fired per ejection burst, and (c) both (a) and (b) in order to modify fluid plume characteristics of fluid ejected from the fluid ejection head (112, 202, 250) through the fluid outlet nozzle (104, 208, 252), and
activating the fluid ejection device (100, 200) to deliver the pharmaceutical drug to a patient.

6. The method of claim 5, wherein the pharmaceutical drug is delivered to the patient with a fluid plume angle ranging from about 25 to about 60 degrees.

7. The method of claim 5, wherein the pharmaceutical drug is delivered to the patient with a fluid plume height ranging from about 10 to about 25 centimeters.

8. The method of claim 5, wherein the pharmaceutical drug is delivered to the patient with a fluid jet length ranging from about 2 to about 10 centimeters from the fluid ejection head (112, 202, 250).

9. The method of claim 5, wherein the pharmaceutical drug is ejected with a plume characteristic that delivers the pharmaceutical drug to a mucosa area of a nasal cavity (10) of the patient.

10. The method of claim 5, wherein the pharmaceutical drug is ejected with a plume characteristic that evenly distributes the pharmaceutical drug throughout a nasal cavity (10) of the patient.

11. The method of claim 5, wherein the pharmaceutical drug is ejected with a plume characteristic that increases a drug dose delivery rate to the patient.

12. A method for nasal cavity injection of pharmaceutical drugs, comprising:
providing a fluid ejection device (100, 200) comprising a cartridge body (102), a fluid outlet nozzle (104, 208, 252) attached to the cartridge body (102) and a fluid jet ejection cartridge (106) containing a pharmaceutical drug disposed in the cartridge body (102), wherein a fluid ejection head (112, 202, 250) is attached to the fluid jet ejection cartridge (106) and the fluid ejection head (112, 202, 250) is in fluid flow communication with the fluid outlet nozzle (104, 208, 252),
providing a processor (114) in electrical communication with the fluid ejection head (112, 202, 250),
configuring the processor (114) to execute a control algorithm to select one or more operating parameters selected from the group consisting of (a) a number of fluid ejectors (214, 216, 218, 220, 222, 254, 256, 258) fired per ejection burst, (b) a position of fluid ejectors (214, 216, 218, 220, 222, 254, 256, 258) fired per ejection burst, and (c) both (a) and (b) in order to modify fluid plume characteristics of fluid ejected from the fluid ejection head (112, 202, 250) through the fluid outlet nozzle (104, 208, 252), and
activating the fluid ejection device (100, 200) to deliver the pharmaceutical drug in the nasal cavity (10) of a person.

13. The method of claim 12, wherein the pharmaceutical drug is ejected with a plume characteristic that delivers the pharmaceutical drug to a mucosa area of the nasal cavity (10).

14. The method of claim 12, wherein the pharmaceutical drug is ejected with a plume characteristic that evenly distributes the pharmaceutical drug throughout the nasal cavity (10).

15. The method of claim 12, wherein the pharmaceutical drug is ejected with a plume characteristic that increases a drug dose delivery rate to the nasal cavity (10).

16. The method of claim 12, wherein the pharmaceutical drug is delivered to the nasal cavity (10) with a fluid plume angle ranging from about 25 to about 60 degrees.

17. The method of claim 12, wherein the pharmaceutical drug is delivered to the nasal cavity (10) with a fluid plume height ranging from about 15 to about 25 centimeters.

18. The method of claim 12, wherein the pharmaceutical drug is delivered to the nasal cavity with a fluid jet length ranging from about 2 to about 10 centimeters from the fluid ejection head (112, 202, 250).
